# EUROPEAN PATENT APPLICATION

(11) **EP 2 842 577 A1**
(43) Date of publication of application: **04.03.2015**
(21) Application number: 14189625.8
(22) Date of filing: 11.03.2011
(51) Int. Cl.: A61K 48/00, A61K 31/7105, A61K 31/7115, A61K 31/712, A61K 31/7125, A61K 35/76, A61P 9/00, A61P 9/04, A61P 9/10

(54) **Method for proliferating cardiomyocytes using micro-RNA**

(30) Priority: 12.03.2010 JP 2010056558
(62) Divisional of application: 11753482.6
(71) Applicant: Daiichi Sankyo Company, Limited, Tokyo 103-8426 (JP)
(72) Inventor: Kawashima, Kayoko, Hyogo, 650-0047 (JP); Koshimizu, Uichi, Hyogo, 650-0047 (JP)
(74) Representative: Vossius & Partner

(57) **Abstract**

The present invention addresses the problems of providing a method for proliferating cardiomyocytes using a miRNA that promotes the proliferation of cardiomyocytes, a vector for use in the treatment of heart disease, a pharmaceutical composition for treating heart disease, and so forth.

The present invention provides a method for proliferating cardiomyocytes using a miRNA having the cardiomyocyte proliferation promoting action, a vector for use in the treatment of heart disease that comprises said miRNA, a pharmaceutical composition for treating heart disease that comprises said vector, and so forth. A particularly preferred miRNA is one that is selected from the group consisting of mature miRNAs, i.e., miR-148a, miR-148b, miR-152, and miR-373, and precursors of said miRNAs, as well as variants and analogs thereof.

SEQUENCE LISTING

<110> DAIICHI SANKYO COMPANY, LIMITED

<120> METHOD FOR PROLIFERATING CARDIOMYOCYTES USING MICRO-RNA

<130> U2655 EP S3

<140> EP 11 75 3482.6
<141> 2011-03-11

<150> PCT/JP2011/055789
<151> 2011-03-11

<150> JP2010-056558
<151> 2010-03-12

<160> 18

<170> PatentIn version 3.1

<210> 1
<211> 8
<212> RNA
<213> Homo sapiens

<400> 1
ucagugca 8

<210> 2
<211> 8
<212> RNA
<213> Homo sapiens

<400> 2
gaagugcu 8

<210> 3
<211> 22
<212> RNA
<213> Homo sapiens

<400> 3
ucagugcacu acagaacuuu gu 22

<210> 4
<211> 68
<212> RNA
<213> Homo sapiens

<400> 4
gaggcaaagu ucugagacac uccgacucug aguaugauag aagucagugc acuacagaac 60

uuugucuc 68

<210> 5
<211> 22
<212> RNA
<213> Homo sapiens

<400> 5

ucagugcauc acagaacuuu gu                                              22

<210>  6
<211>  99
<212>  RNA
<213>  Homo sapiens

<400>  6
caagcacgau uagcauuuga ggugaaguuc uguuauacac ucaggcugug gcucucugaa      60

agucagugca ucacagaacu uugucucgaa agcuuucua                             99

<210>  7
<211>  21
<212>  RNA
<213>  Homo sapiens

<400>  7
ucagugcaug acagaacuug g                                                21

<210>  8
<211>  87
<212>  RNA
<213>  Homo sapiens

<400>  8
uguccccccc ggcccagguu cugugauaca cuccgacucg ggcucuggag cagucagugc      60

augacagaac uugggcccgg aaggacc                                          87

<210>  9
<211>  23
<212>  RNA
<213>  Homo sapiens

<400>  9
gaagugcuuc gauuuugggg ugu                                              23

<210>  10
<211>  69
<212>  RNA
<213>  Homo sapiens

<400>  10
   gggauacuca aaaugggggc gcuuuccuuu uugucuguac ugggaagugc uucgauuuug      60
   gggugaccc                                                             69

<210>  11
<211>  27
<212>  DNA
<213>  Artificial

<220>
<223>  Forward primer for amplifying coding region of miR-148a gene.

<400>  11
caccgaacac acctgcagga agaaact                                          27

<210>  12
<211>  25
<212>  DNA
<213>  Artificial

<220>
<223>  Reverse primer for amplifying coding region of miR-148a gene.

<400>  12
gttcccattt acagggttta accca                                          25


<210>  13
<211>  26
<212>  DNA
<213>  Artificial

<220>
<223>  Forward primer for amplifying coding region of miR-152 gene.

<400>  13
caccgtccca gactcggctc ccatca                                         26


<210>  14
<211>  22
<212>  DNA
<213>  Artificial

<220>
<223>  Reverse primer for amplifying coding region of miR-152 gene.

<400>  14
actcgaggtg gacaccctgt gt                                             22


<210>  15
<211>  25
<212>  DNA
<213>  Artificial

<220>
<223>  Forward primer for amplifying coding region of miR-373 gene.

<400>  15
caccgtgacc aaggggctgt atgca                                          25


<210>  16
<211>  22
<212>  DNA
<213>  Artificial

<220>
<223>  Reverse primer for amplifying coding region of miR-373 gene.

<400>  16
ctgcccaccc cagaatatgc ca                                             22


<210>  17
<211>  23
<212>  DNA
<213>  Artificial

<220>
<223>  Forward primer for amplifying coding region of miR-141 gene.

<400>  17
caccgcaggg atcctgggcc tga                                              23


<210>  18
<211>  22
<212>  DNA
<213>  Artificial

<220>
<223>  Reverse primer for amplifying coding region of miR-141 gene.

<400>  18
cgggaagaca atggaggtgc ct                                               22

## Description

### TECHNICAL FIELD

The present invention relates to a method for proliferating cardiomyocytes using microRNAs, a vector for treating heart disease, and a pharmaceutical composition for treating heart disease.

### BACKGROUND ART

In adults, cardiomyocytes have lost their ability to divide, so once they become necrotic or lost entirely upon exposure to various stresses such as ischemia or myocarditis, the lost cardiomyocytes can be never regenerated. In consequence, the remaining cardiomyocytes try to maintain the cardiac function through compensatory hypertrophy, but if this condition continues and exceeds tolerable threshold of the myocardial tissue, it leads to further exhaustion and death of cardiomyocytes and a consequent impairments myocardial function, namely, heart failure.

Heart diseases including heart failure are the second most common cause of death among Japanese people. In addition, patients with heart disease have a very poor prognosis and their five year survival rate is only about 50%. Hence, the development of an effective therapy of heart failure is considered to be extremely beneficial from the viewpoints of medical welfare and medical economy.

Conventionally used therapeutics for heart failure are cardiotonic drugs such as digitalis and xanthine preparations that increase the force of myocardial contraction but these drugs are known to potentially worsen rather than ameliorate the diseased condition if administered over a prolonged period of time. In recent years, treatments with drugs such as β-blockers and ACE inhibitors that reduce an excessive cardiac load due to elevations of the sympathetic nervous system or rennin-angiotensin system are becoming the mainstream but these methods are just symptomatic and unable to restore the damaged cardiac tissue itself.

Although heart transplantation is a radical therapy for severe heart failure, it involves various problems such as the scarcity of donor organs, sensitive medical ethics, and the high costs of physical or economic burdens on patients, making it difficult to take this approach as a common therapy.

MicroRNAs (hereinafter sometimes designated as miRNAs) are non-coding DNAs of 21-25 bases in length that occur within cells. They are conserved widely ranging from nematodes to mammals and recently are attracting attention as molecules that play an important role in regulating the expression of various genes *in vivo.*

At first, a miRNA is transcribed as a primary miRNA transcript (pri-miRNA) of approximately several hundred to several thousand bases from a miRNA gene on the genomic DNA. Subsequently, the pri-miRNA is processed in the nucleus with an RNase III enzyme called Drosha to form a precursor miRNA (pre-miRNA) of approximately 70 bases that has a hairpin structure. Thereafter, by means of Exportin-5, the pre-miRNA is transported from the nucleus to the cytoplasm, where it is processed with an RNase III enzyme called Dicer to form double-stranded mature miRNAs of 21-25 bases. It is known that one of the two strands of the mature miRNA forms a complex with the protein called an RNA induced suppressor complex (RISC) and binds the mRNA of a target gene having a complementary sequence to thereby suppress the expression of the target gene (Non-Patent Documents 1 and 2.)

It is speculated that approximately 1000 miRNAs exist per biological species, each being considered to regulate the expression of more than one target gene, eventually controlling about one third of the protein-coding genome (Non-Patent Document 3.) In addition, miRNAs have been shown to be involved in biological reactions such as development, differentiation and viral infection, and their relationship with human diseases is also suggested. Particularly for the relationship between cancer and miRNA, the existence of many miRNAs that display different expression modes between a normal tissue and cancerous tissue suggests that miRNAs are strongly implicated in the carcinogenesis process, and the existence of two types of miRNA, a carcinogenesis promoter and a tumor suppressor, has been reported (Non-Patent Documents 4, 5, and 6.)

In connection with heart diseases, miRNA expression profiling analyses in a normal heart and a diseased heart have been reported by many research groups. In some of the reports, specific functions were analyzed to give observations that the expression of miR-195 increased in cardiac hypertrophy, dilating the heart tissues and triggering dilated cardiomyopathy or heart failure in transgenic mice that had been subjected to forced expression of said miRNA (Patent Document 1 and Non-Patent Document 7) and that miR-1, the miRNA characterized by decreased expression in cardiac hypertrophy, suppressed the proliferation of cardiomyocytes (Patent Document 2 and Non-Patent Document 8.)

### CITATION LIST

### PATENT DOCUMENTS

Patent Document 1: WO 2009/062169
Patent Document 2: WO 2006/107826

### NON-PATENT DOCUMENTS

Non-Patent Document 1: He & Hannon, Nature Reviews Genetics, 5: 522-531, (2004)
Non-Patent Document 2: Bartel, Cell, 116:281-297, (2004)
Non-Patent Document 3 : Berezikov et al., Cell, 120:21-24, (2005)
Non-Patent Document 4 : Esquela-Kerscher & Slack, Nature Reviews Cancer, 6:259-269, (2006)
Non-Patent Document 5 : Kent & Mendell, Oncogene, 25:6188-6196, (2006)
Non-Patent Document 6: Zhang et al., Developmental Biology, 302:1-12, (2007)
Non-Patent Document 7: van Rooij et al., Proceedings of the National Academy of Sciences of the United States of America, 103:18255-18260, (2006)
Non-Patent Document 8: Zhao et al., Cell, 129: 303-317, (2007)

### SUMMARY OF INVENTION

### TECHNICAL PROBLEMS

The present invention addresses the problems of providing a method for proliferating cardiomyocytes which comprises identifying a miRNA that promotes the proliferation of cardiomyocytes and using said miRNA, a vector for use in the treatment of heart disease, a pharmaceutical composition for treating heart disease, and so forth.

### SOLUTION TO PROBLEMS

To solve these problems, the present inventors noted microRNAs (hereinafter sometimes designated miRNAs) as a factor regulating the proliferation of cardiomyocytes and made a study involving analyses about miRNAs regulating the proliferation of cardiomyocytes. As a result of their intensive study, the present inventors successfully specified a plurality of miRNAs regulating the proliferation of cardiomyocytes, which has led to the accomplishment of the present invention.

In a first embodiment of the present invention, the present inventors provide miRNAs having an action for promoting the proliferation of cardiomyocytes. More specifically, the miRNAs of the present invention encompass the following embodiments:
(1-1) A miRNA having a cardiomyocyte proliferation promoting action;
(1-2) The miRNA as recited in (1-1) above, which is a substance selected from the group consisting of a mature miRNA, a precursor of said miRNA, as well as variants and analogs thereof;
(1-3) The miRNA as recited in (1-1) or (1-2) above, which is a substance comprising a seed sequence depicted by SEQ ID NO: 1 or SEQ ID NO: 2;
(1-4) The miRNA as recited in (1-3) above, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof;
(1-5) The miRNA as recited in (1-3) above, wherein the substance comprising the seed sequence depicted by SEQ ID NO: 2 is a substance selected from the group consisting of miR-373 (SEQ ID NO: 9), precursors thereof, as well as variants and analogs thereof.

In a second embodiment of the present invention, the present inventors provide an invention that relates to pharmaceutical uses of the miRNA having a cardiomyocyte proliferation promoting action and which can be described as (a) a pharmaceutical composition for treating heart disease using a nucleic acid that encodes the above-mentioned miRNA having a cardiomyocyte proliferation promoting action; (b) a method of treating heart disease wherein a nucleic acid that encodes the miRNA having a cardiomyocyte proliferation promoting action is introduced into a damaged site in the heart of an individual to thereby proliferate cardiomyocytes at said site; (c) use of a nucleic acid that encodes the miRNA having a cardiomyocyte proliferation promoting action for the manufacture of pharmaceuticals for the treatment of heart disease, or (d) a nucleic acid that encodes the miRNA having a cardiomyocyte proliferation promoting action for use in the treatment of heart disease. As used herein, the term "nucleic acid that encodes the miRNA" refers to (i) a mature miRNA, a precursor of said miRNA, as well as variants and analogs thereof, or (ii) an expression vector for expressing a mature miRNA, a precursor of said miRNA, as well as variants and analogs thereof either *in vivo* or within cells.

To describe the pharmaceutical composition as invention (a) more specifically, it encompasses the following embodiments:
(2-a-1) A pharmaceutical composition for the treatment of heart disease comprising a nucleic acid that encodes the miRNA having a cardiomyocyte proliferation promoting action;
(2-a-2) The pharmaceutical composition as recited in (2-a-1) above, wherein the miRNA is a substance selected from the group consisting of a mature miRNA, a precursor of said miRNA, as well as variants and analogs thereof;
(2-a-3) The pharmaceutical composition as recited in (2-a-1) or (2-a-2) above, wherein the miRNA is a substance comprising a seed sequence depicted by SEQ ID NO: 1 or SEQ ID NO: 2;
(2-a-4) The pharmaceutical composition as recited in (2-a-3) above, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof;
(2-a-5) The pharmaceutical composition as recited in (2-a-3) above, wherein the substance comprising the seed sequence depicted by SEQ ID NO: 2 is a substance selected from the group consisting of miR-373 (SEQ ID NO: 9), precursors thereof, as well as variants and analogs thereof;
(2-a-6) The pharmaceutical composition as recited in any one of (2-a-1) to (2-a-5) above, wherein the nucleic acid that encodes the miRNA having a cardiomyocyte proliferation promoting action is contained as an expression vector or a liposome for introduction and expression in cardiomyocytes;
(2-a-7) The pharmaceutical composition as recited in (2-a-6) above, wherein the vector is a viral vector or a non-viral expression vector;
(2-a-8) The pharmaceutical composition as recited in (2-a-7) above, wherein the viral vector is selected from the group consisting of adenoviral vectors, adeno-associated viral (AAV) vectors, retroviral vectors, hemagglutinating virus of Japan (HVJ, also known as Sendai virus) vector, lentiviral vectors, vaccinia viral vectors, chick poxviral vectors, and papovaviral vectors;
(2-a-9) The pharmaceutical composition as recited in any one of (2-a-1) to (2-a-8) above, wherein the miRNA is a miRNA derivative comprising at least one modified internucleoside linkage, at least one modified sugar moiety, at least one modified base, or any combinations thereof;
(2-a-10) The pharmaceutical composition as recited in any one of (2-a-1) to (2-a-9) above, wherein the heart disease is myocardial infarction, ischemic cardiac disease, congestive heart failure, hypertrophic cardiomyopathy, dilated cardiomyopathy, myocarditis, or chronic heart failure.

To describe the therapeutic method as invention (b) more specifically, it encompasses the following embodiments:
(2-b-1) A method for the treatment of heart disease wherein a nucleic acid that encodes the miRNA having a cardiomyocyte proliferation promoting action is introduced into a damaged site in the heart of an individual to thereby proliferate cardiomyocytes at said site;
(2-b-2) The method as recited in (2-b-1) above, wherein the miRNA is a substance selected from the group consisting of a mature miRNA, a precursor of said miRNA, as well as variants and analogs thereof;
(2-b-3) The method as recited in (2-b-1) or (2-b-2) above, wherein the miRNA is a substance comprising a seed sequence depicted by SEQ ID NO: 1 or SEQ ID NO: 2;
(2-b-4) The method as recited in (2-b-3) above, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof;
(2-b-5) The method as recited in (2-b-3) above, wherein the substance comprising the seed sequence depicted by SEQ ID NO: 2 is a substance selected from the group consisting of miR-373 (SEQ ID NO: 9), precursors thereof, as well as variants and analogs thereof;
(2-b-6) The method as recited in any one of (2-b-1) to (2-b-5) above, wherein the nucleic acid that encodes the miRNA having a cardiomyocyte proliferation promoting action is introduced into cardiomyocytes by means of an expression vector or a liposome;
(2-b-7) The method as recited in (2-b-6) above, wherein the vector is a viral vector or a non-viral expression vector;
(2-b-8) The method as recited in (2-b-7) above, wherein the viral vector is selected from the group consisting of adenoviral vectors, adeno-associated viral (AAV) vectors, retroviral vectors, hemagglutinating virus of Japan (HVJ, also known as Sendai virus) vector, lentiviral vectors, vaccinia viral vectors, chick poxviral vectors, and papovaviral vectors;
(2-b-9) The method as recited in any one of (2-b-1) to (2-b-8) above, wherein the miRNA is a miRNA derivative comprising at least one modified internucleoside linkage, at least one modified sugar moiety, at least one modified base, or any combinations thereof;
   (2-b-10) The method as recited in any one of (2-b-1) to (2-b-9) above, wherein the heart disease is myocardial infarction, ischemic cardiac disease, congestive heart failure, hypertrophic cardiomyopathy, dilated cardiomyopathy, myocarditis, or chronic heart failure.

To describe the use as invention (c) more specifically, it encompasses the following embodiments:
(2-c-1) Use of a nucleic acid that encodes the miRNA having a cardiomyocyte proliferation promoting action for the manufacture of pharmaceutical compositions for the treatment of heart disease;
(2-c-2) The use as recited in (2-c-1) above, wherein the miRNA is a substance selected from the group consisting of a mature miRNA, a precursor of said miRNA, as well as variants and analogs thereof;
(2-c-3) The use as recited in (2-c-1) or (2-c-2) above, wherein the miRNA is a substance comprising a seed sequence depicted by SEQ ID NO: 1 or SEQ ID NO: 2;
(2-c-4) The use as recited in (2-c-3) above, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof;
(2-c-5) The use as recited in (2-c-3) above, wherein the substance comprising the seed sequence depicted by SEQ ID NO: 2 is a substance selected from the group consisting of miR-373 (SEQ ID NO: 9), precursors thereof, as well as variants and analogs thereof;
(2-c-6) The use as recited in any one of (2-c-1) to (2-c-5) above, wherein the nucleic acid that encodes the miRNA having a cardiomyocyte proliferation promoting action is contained as an expression vector or a liposome for introduction and expression in cardiomyocytes;
(2-c-7) The use as recited in (2-c-6) above, wherein the vector is a viral vector or a non-viral expression vector;
(2-c-8) The use as recited in (2-c-7) above, wherein the viral vector is selected from the group consisting of adenoviral vectors, adeno-associated viral (AAV) vectors, retroviral vectors, hemagglutinating virus of Japan (HVJ, also known as Sendai virus) vector, lentiviral vectors, vaccinia viral vectors, chick poxviral vectors, and papovaviral vectors;
(2-c-9) The use as recited in any one of (2-c-1) to (2-c-8) above, wherein the miRNA is a miRNA derivative comprising at least one modified internucleoside linkage, at least one modified sugar moiety, at least one modified base, or any combinations thereof;
(2-c-10) The use as recited in any one of (2-c-1) to (2-c-9) above, wherein the heart disease is myocardial infarction, ischemic cardiac disease, congestive heart failure, hypertrophic cardiomyopathy, dilated cardiomyopathy, myocarditis, or chronic heart failure.

To describe the nucleic acid as invention (d) more specifically, it encompasses the following embodiments:
(2-d-1) A nucleic acid that encodes the miRNA having a cardiomyocyte proliferation promoting action for use in the treatment of heart disease;
(2-d-2) The nucleic acid as recited in (2-d-1) above, wherein the miRNA is a substance selected from the group consisting of a mature miRNA, a precursor of said miRNA, as well as variants and analogs thereof;
(2-d-3) The nucleic acid as recited in (2-d-1) or (2-d-2) above, wherein the miRNA is a substance comprising a seed sequence depicted by SEQ ID NO: 1 or SEQ ID NO: 2;
(2-d-4) The nucleic acid as recited in (2-d-3) above, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof;
(2-d-5) The nucleic acid as recited in (2-d-3) above, wherein the substance comprising the seed sequence depicted by SEQ ID NO: 2 is a substance selected from the group consisting of miR-373 (SEQ ID NO: 9), precursors thereof, as well as variants and analogs thereof;
(2-d-6) The nucleic acid as recited in any one of (2-d-1) to (2-d-5) above, wherein the nucleic acid that encodes the miRNA having a cardiomyocyte proliferation promoting action is contained as an expression vector or a liposome for introduction and expression in cardiomyocytes;
(2-d-7) The nucleic acid as recited in (2-d-6) above, wherein the vector is a viral vector or a non-viral expression vector;
(2-d-8) The nucleic acid as recited in (2-d-7) above, wherein the viral vector is selected from the group consisting of adenoviral vectors, adeno-associated viral (AAV) vectors, retroviral vectors, hemagglutinating virus of Japan (HVJ, also known as Sendai virus) vector, lentiviral vectors, vaccinia viral vectors, chick poxviral vectors, and papovaviral vectors;
(2-d-9) The nucleic acid as recited in any one of (2-d-1) to (2-d-8) above, wherein the miRNA is a miRNA derivative comprising at least one modified internucleoside linkage, at least one modified sugar moiety, at least one modified base, or any combinations thereof; (2-d-10) The nucleic acid as recited in any one of (2-d-1) to (2-d-9) above, wherein the heart disease is myocardial infarction, ischemic cardiac disease, congestive heart failure, hypertrophic cardiomyopathy, dilated cardiomyopathy, myocarditis, or chronic heart failure.

In a third embodiment of the present invention, the present inventors provide a method for proliferating cardiomyocytes using the above-described nucleic acid that encodes the miRNA having a cardiomyocyte proliferation promoting action. More specifically, this method for proliferating cardiomyocytes encompasses the following embodiments:
(3-1) A method for proliferating cardiomyocytes wherein a nucleic acid that encodes the miRNA having a cardiomyocyte proliferation promoting action is introduced and expressed in cardiomyocytes;
(3-2) The method as recited in (3-1) above, wherein the miRNA is a substance selected from the group consisting of a mature miRNA, a precursor of said miRNA, as well as variants and analogs thereof;
(3-3) The method as recited in (3-1) or (3-2) above, wherein the miRNA is a substance comprising a seed sequence depicted by SEQ ID NO: 1 or SEQ ID NO: 2;
(3-4) The method as recited in (3-3) above, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof;
(3-5) The method as recited in (3-3) above, wherein the substance comprising the seed sequence depicted by SEQ ID NO: 2 is a substance selected from the group consisting of miR-373 (SEQ ID NO: 9), precursors thereof, as well as variants and analogs thereof;
(3-6) The method as recited in any one of (3-1) to (3-5) above, wherein the nucleic acid that encodes the miRNA having a cardiomyocyte proliferation promoting action is introduced into cardiomyocytes using an expression vector or a liposome;
(3-7) The method as recited in (3-6) above, wherein the vector is a viral vector or a non-viral expression vector;
(3-8) The method as recited in (3-7) above, wherein the viral vector is selected from the group consisting of adenoviral vectors, adeno-associated viral (AAV) vectors, retroviral vectors, hemagglutinating virus of Japan (HVJ, also known as Sendai virus) vector, lentiviral vectors, vaccinia viral vectors, chick poxviral vectors, and papovaviral vectors;
(3-9) The method as recited in any one of (3-1) to (3-8) above, wherein the miRNA is a miRNA derivative comprising at least one modified internucleoside linkage, at least one modified sugar moiety, at least one modified base, or any combinations thereof.

### ADVANTAGEOUS EFFECT OF INVENTION

By using the miRNAs according to the present invention, one can provide a method for proliferating cardiomyocytes, a vector for use in the treatment of heart disease, and a pharmaceutical composition for treating heart disease. When the method of proliferating cardiomyocytes according to the present invention is used, cell division of cardiomyocytes can be induced for cell proliferation to take place more efficienly than in the prior art, and the cardiomyocyes prepared by this method can be utilized as cells for the screening of various drugs and for transplantation therapy. In addition, by applying this method in gene therapy, heart diseases due to necrosis, depletion or other failure of cardiomyocytes may potentially be treated in an attempt as part of regenerative therapy. The miRNAs according to the present invention have been shown to exhibit a marked proliferation activity on cardiomyocytes.

### BREIF DESCRIPTION OF DRAWINGS

FIG. 1 is a diagram showing the proportions of Ki67 positive cardiomyocytes in primary cultures of cardiomyocyes transfected with miR-148a (SEQ ID NO: 3), miR-152 (SEQ ID NO: 7), and miR-373 (SEQ ID NO: 9) in Example 2; Ki67 is a cell nucleoprotein and used as a marker for detecting proliferating cells.
FIG 2 is a diagram showing the proportions of Ki67 positive cardiomyocytes in primary cultures of cardiomyocyes transfected with recombinant adenoviruses expressing miR-148a (SEQ ID NO: 3), miR-152 (SEQ ID NO: 7), and miR-373 (SEQ ID NO: 9) in Example 3; *"None"* refers to untreated samples.
FIG 3-1 is a diagram showing the proportions of phosphorylated histone H3 (H3P) positive cardiomyocytes in primary cultures of cardiomyocyes transfected with miR-148a (SEQ ID NO: 3), miR-152 (SEQ ID NO: 7), and miR-373 (SEQ ID NO: 9) in Example 4.
FIG.3-2 is a diagram showing the proportions of H3P positive cardiomyocytes in primary cultures of cardiomyocyes transfected with recombinant adenoviruses expressing miR-148a (SEQ ID NO: 3), miR-152 (SEQ ID NO: 7), and miR-373 (SEQ ID NO: 9) in Example 4; *"None"* refers to untreated samples.
FIG.3-3 is a set of micrographs showing primary cultures of cardiomyocyes that were transfected with recombinant adenoviruses expressing miR-148a (SEQ ID NO: 3), miR-152 (SEQ ID NO: 7), and miR-373 (SEQ ID NO: 9) and then stained with an anti-H3P antibody, an anti-Troponin T antibody, and DAPI in Example 4; H3P (green),Troponin T (red), and DAPI (blue) are depicted.
FIG 4 is a diagram showing the proportions of Ki67 positive cardiomyocytes in tissue sections of the hearts of rats transfected *in vivo* with recombinant adenoviruses expressing miR-148a (SEQ ID NO: 3), miR-152 (SEQ ID NO: 7), and miR-373 (SEQ ID NO: 9) in Example 5.

### DESCRIPTION OF EMBODIMENT

When carrying out the present invention, skilled artisans, if they want to know more about general methods and prior art in connection with molecular biology, microbiology, cell biology, recombinant DNA technology, etc., may, unless otherwise noted, look at standard reference books in those fields. Examples that can be referred to include: Molecular Cloning: A Laboratory Manual, 3rd Edition (Sambrook & Russell, Cold Spring Harbor Laboratory Press, 2001); Current Protocols in Molecular biology (ed. by Ausubel et. al, John Wiley & Sons, 1987); Methods in Enzymology Series (Academic Press); PCR Protocols: Methods in Molecular Biology (ed. by Bartlett & Striling, Humana Press, 2003); Animal Cell Culture: A Practical Approach, 3rd Edition (ed. by Masters, Oxford University Press, 2000); Antibodies: A Laboratory Manual (ed. by Harlow et al. & Lane, Cold Spring Harbor Laboratory Press, 1987.) Reagents for the cell culture and cell biological experiments that are referred to herein are on the commercial market (for example, from Sigma, Aldrich, Invitrogen/GIBCO, Clontech, Stratagene, etc.) and are readily available.

In its first embodiment, the present invention provides miRNAs having an action for promoting the proliferation of cardiomyocytes.

In its second embodiment, the present invention provides a pharmaceutical composition for treating heart disease using a nucleic acid that encodes the above-mentioned miRNA having a cardiomyocyte proliferation promoting action; a method of treating heart disease wherein a nucleic acid that encodes the miRNA having a cardiomyocyte proliferation promoting action is introduced into a damaged site in the heart of an individual to thereby proliferate cardiomyocytes at said site; use of a nucleic acid that encodes the miRNA having a cardiomyocyte proliferation promoting action for the manufacture of pharmaceuticals for the treatment of heart disease, and a nucleic acid that encodes the miRNA having a cardiomyocyte proliferation promoting action for use in the treatment of heart disease.

In its third embodiment, the present invention provides a method for proliferating cardiomyocytes using the above-described nucleic acid that encodes the miRNA having a cardiomyocyte proliferation promoting action.

### Cardiomyocytes

The cardiomyocytes to be expanded by the method according to the present invention may generally encompass those at all stages of development, i.e., adult cardiomyocytes, juvenile cardiomyocytes, and embryonic cardiomyocytes. The direct target in the present invention may be cardiomyocytes that are found in mammalian heart tissues but in the first and third embodiments of the present invention, *ex vivo* cultured cardiomyocytes and the like may also be used.

The *ex vivo* cultured cardiomyocytes that can be used in the present invention may originate from any supply sources, including not only cardiomyocytes isolated from the *in vivo* heart tissue by various methods such as enzymatic treatment but also their primary cultures obtained by culturing them under suitable conditions for about 1 to 5 days. Specific methods of culturing cardiomyocytes are described in many reference papers and representative methods that can be used include Chien's method and its modifications (Chien et al., The Journal of Clinical Investigation, 75:1770-1780, (1985) ; Meidell et al., American Journal of Physiology, 251:H1076-H1084, (1986) ; Tamamori et al., American Journal of Physiology, 275:H2036-H2040, (1998)).

The cultured cardiomyocytes that can be used are by no means limited to the above examples and may also include cardiomyocytes obtained by induced differentiation from various types of stem cells. The stem cells to be used in carrying out the present invention are cells having such an ability that they can differentiate into cells having cardiomyocyte-like phenotypes under *in vitro* culutre conditions and specific examples include pluripotent stem cells that are already in extensive use as culutured cells and which are exemplified by embryonic stem cells (ES cells), induced pluripotent stem cells (iPS cells) and embryonic germ cells (EG cells) that are derived from mammals such as mouse, monkey and human. For the methods of preparing, passaging and preserving those cells, standard protocols have already been established and skilled artisans may find it easy to use these pluripotent stem cells by referring to a plurality of reference books such as Manipulating the Mouse Embryo : A laboratory manual (ed. by Hogan et al., Cold Spring Harbor Laborayory Press, 1994) and Embryonic Stem Cells (ed. by Turksen, Humana Press, 2002), as well as a plurality of reference papers (Matsui et al., Cell, 70:841-847, (1992) ; Shamblott et al., Proceedings of the National Academy of Sciences of the United States of America, 95:13726-13731, (1998); Okita et al., Nature, 448:313-317, (2007) ; Takahashi et al., Cell, 131:861-872, (2007)). The stem cells that can be used in the present invention are by no means limited to the aforementioned stem cells and any stem cells can be used as long as they have traits similar to those of ES cells. The characteristics similar to those of ES cells as referred to above can be defined by cell biological properties specific for ES cells, as exemplified by the presence of a surface (antigen) marker specific for ES cells, the expression of an ES cell specific gene, and even the abilities to form teratoma and chimeric mouse.

Even in the case of cells that do not have property similar to those of ES cells or which are not pluripotent stem cells, the method described herein can be applied if they are cells at least having such an ability that they can differentiate into cells having cardiomyocyte-like phenotypes under *in vitro* culutre conditions. Examples of such cells may include bone marrow mesenchymal stem cells (Bruder et al., USP 5,736,396 ; Pittenger et al., Science, 284:143-147, (1999), CMG clls also derived from bone marrow (Makino et al., The Journal of Clinical Investigation, 103:697-705, (1999) ; WO01/048151), and Spoc cells derived from muscle tissue (WO03/035382).

Culture methods that can be used in the present invention to prepare cardiomyocytes from stem cells may be of any types that are suitable for induced differentiation into cardiomyocytes. A plurality of specific methods for induced differentiation have already been established and any skilled artisan can induce the differentation of stem cells into cardiomyocytes by referring to such reference books as Embryonic Stem Cells (ed. by Turksen, Humana Press, 2002) and a plurality of reference papers (Klug et al., The Journal of Clinical Investigation, 98:216-224, (1996); Wobus et al., Journal of Molecular and Cellular Cardiology, 29:1525-1539, (1997); Kehat et al., The Journal of Clinical Investigation, 108:407-414, (2001); Xu et al., Circulation Research, 91:501-508, (2002); Takahashi et al., Circulation, 107:1912-1916, (2003); Field et al., USP 6,015,671; Xu et al., WO03/06950).

### MicroRNAs (miRNAs)

The present invention provides microRNAs (miRNAs) having an action for promoting the proliferation of cardiomyocytes. The miRNAs according to the present invention may be of any types as long as they promote the proliferation of cardiomyocytes and they can be used to proliferate the cardiomyocytes described above.

The miRNAs as referred to in the present invention are small non-protein coding RNAs that are most abundantly expressed in eukaryotes including mammals and are considered to play a role in various biological phenomena including development, differentiation, and proliferation. The miRNAs that can be used in the present invention include mature miRNAs, precursors of said miRNAs, as well as variants and analogs thereof. The miRNAs according to the present invention may be single- or double-stranded.

The mature miRNAs as referred to in the present invention are miRNAs that have been completely processed and they are 15 to 30 nucleotides long (mostly 21 to 25 nucleotides long). Mature miRNAs can make up a miRNA family or a group of miRNAs that share at least six consecutive nucleotides among the 1 to 12 nucleotides on the 5' side of the mature miRNA molecule (said shared nucleotide portion is also called a "seed region" (Brennecke et al., PLoS Biology, 3:e85, (2005)). Hence, a miRNA family contains a plurality of mature miRNAs that have an identical 5' region but are different on the 3' side of the mature miRNA. Members of the same miRNA family have the same function even if their nucleic acid sequences in the 3' region are different, as has been reported for the sequence of an 8-mer seed region (seed sequence) at a single site in Bagpipe 3'UTR (Brennecke et al., PLoS Biology, 3:e85, (2005)) and in this reported case, two different miRNAs among those of a miRNA family that share in the 5' region a sequence complementary to the sequence that binds the sequence of the aforementioned 8-mer seed region at a single site were different on the 3' side and yet they could suppress the expression of the reporter gene including the 8-mer target.

The precursors of miRNAs are precursor RNAs of mature miRNAs. The process of preparing a mature miRNA in a living cell proceeds as follows: at first, a miRNA is transcribed as a primary miRNA transcript (pri-miRNA) of approximately several hundred to several thousand bases from a miRNA gene on the genomic DNA; subsequently, the pri-miRNA is processed in the nucleus with an RNase III enzyme called Drosha to form a precursor miRNA (pre-miRNA) of approximately 70 bases that has a hairpin structure. Hence, the precursors of miRNA as referred to in the present invention include both the primary miRNA transcript (pri-miRNA) and the precursor miRNA (pre-miRNA).

The miRNA variants are miRNAs in which one or several nucleotides are deleted, substituted, inserted or added in comparison with the mature miRNAs or their precursors that are inherent in organisms. Here, the term "several" typically means up to 7 or 6, preferably up to 5 or 4, and more preferably up to 3 to 1. As noted above, a "seed region" exists on the 5' side of each of the mature miRNA molecules that make up a miRNA family and any members of the same miRNA family have the same function even if they differ in the nucleic acid sequence of the region on the 3' side. Hence, miRNA variants are preferably such miRNAs that nucleotides are deleted, substituted, inserted or added in areas other than the seed region of a mature miRNA.

The miRNA analogs are miRNAs that have been so prepared as to mimic endogenous mature miRNAs or their precursors. These substances are on the commercial market (for example, from Ambion) and readily available.

In order to enhance their stability, the miRNAs of the present invention may be used in such forms as nucleic acid derivatives and/or nucleic acids having modified internucleoside linkages. Suitable nucleic acid derivatives can be appropriately selected from the group consisting of known types. Specific examples include nucleic acids containing internucleoside linkages such as methylphosphonate, phosphorothioate, phosphorodithioate, phosphoramidate, phosphotriester, sulfone, siloxane, carbonate, carboxymethylester, acetamidate, carbamate, thioether, bridged phosphoramidate, bridged methylene phosphonate, bridged phosphorothioate, dimethylene-sulfide, dimethylene-sulfoxide, dimethylene-sulfone, 2'-O-alkyl), and 2'-deoxy-2'-fluorophosphorothioate (Uhlmann & Peyman, Chemical Reviews, 90:543-584, (1990); Schneider et al., Tetrahedron Letters, 31:335-338, (1990)). Further examples include derivatives such as Locked Nucleic Acid (Koshkin et al., Journal of the American Chemical Society, 120:13252-13253 (1998) and ENA (US7335765). These nucleic acid derivatives and/or miRNAs having modified internucleoside linkages are referred to as miRNA derivatives in the present invention. The miRNA derivatives include derivatives of mature miRNAs, derivatives of miRNA precursors, derivatives of miRNA variants, and derivatives of miRNA analogs.

The miRNAs that can be used in the present invention may be obtained by using known techniques for isolation from natural products, for chemical synthesis, or for gene recombinant technology-based production; alternatively, they may be available as commercial products. For example, mature miRNAs have been registered in the Sanger miRbase and all the registered miRNAs are available from Ambion, Qiagen, Sigma, Thermo Scientific, and other sources.

Methods for intracellular delivery of miRNAs into *in vivo* cells or *in vitro* cells may be any one of the gene delivery means that are commonly used in the technical art contemplated by the present invention, as exemplified by expressin vectors or liposomes. The above-described mature miRNAs or miRNA precursors may be used in the form of oligonucleic acids or derivatives but from the viewpoints of such aspects as the efficiency of their expression and the duration over which their effect will be sustained, they are preferably used as incorporated into expression vectors.

If an expression vector is to be used as a gene delivery means, it may be of any type as long as it has a promoter that is capable of expressing miRNA, and both a viral expression vector and a non-viral expressin vector may be used.

Exemplary viral expression vectors are those that are derived from adenovirus, adeno-associated virus (AAV), retrovirus, hemagglutinating virus of Japan (HVJ, also known as Sendai virus), lentivirus, vaccinia virus, chick poxvirus, and papovavirus typified by SV40; preferably, adenoviral vectors, AAV vectors, HVJ vector, or lentiviral vectors are used, contributing to efficient gene transfer as well as high-level expression of the transferred gene.

Gene transfer by these viral vectors is one of the most powerful ways to introduce genes into mammalian cells and can be used for gene transfer into virtually all kinds of human cells and many non-human cells.

Since infection with those viruses does not depend on cell cycle, genes can be expressed in various primary culture cell lines and transformed cell lines. Notably, efficient gene transfer is possible even in cells such as cardiomyocytes that do not undergo DNA synthesis or cell division. Since many cells receive more than one copy of recombinant DNA (or RNA), the transferred gene will be temporarily expressed in high level.

In the case of adenoviral vectors, HVJ vectors and the like, DNAs and RNAs usually reside in the cytoplasm and will not be taken up by the nucleus. Hence, another advantage of using these viral vectors is that there is little chance for the occurrence of mutation-inducing errors which might take place randomly when an exogenous gene is incorporated into a host cell genome.

Adenoviral vectors that may be used in one preferred embodiment of the present invention can be prepared by the method using homologous recombination in the host human embryonic kidney 293 cell or *E. coli* (Miyake et al., Proceedings of the National Academy of Sciences of the United States of America, 93:1320-1324, (1996)) or by the simple *in vitro* ligation based method (Mizuguchi & Kay, Human Gene Therapy, 9:2577-2583, (1998)).

Adenoviruses are one of the DNA viruses having a double-stranded DNA genome and human types 5 and 2 are the best studied adenoviruses. By deleting the greater part of E1 and E3 genes in these wild-type adenoviruses, viral vectors having no replicating ability can be prepared and used to insert exogenous DNAs of several kilobases without exerting any harmful action on the formation of virus particles. These recombinant adenoviruses lack the E1 gene which is a transcription regulatory factor but on account of the inserted, gene-specific transcription unit, only the inserted gene of interest can be expressed without depending on the proliferation of the target cell or irrespective of whether other viral genes exist.

Adeno-associated viruses (AAV) are single-stranded DNA viruses of about 4.7 kb that belong to the family *Parvoviridae* and twelve serotypes (1 to 12) of AAV are known. An AAV vector can be prepared by substituting the gene of interest for Rep (involved in replication or genomic incorporation) and Cap (capsid) located between the ITR (inverted terminal repeats) at opposite ends. This vector is introduced into a 293 cell together with a Rep and Cap expressing plasmid and, subsequently, transfection is performed using an adenovirus as a helper virus, to thereby prepare a recombinant adeno-associated viral vector that is accumulated in the nucleus. Recently, a method has been developed that eliminates the need for the helper virus and it involves introducing an E2A, E4 and VA expressing plasmid into an E1A and E1B expressing 293 cells.

Sendai virus (SeV) is a rodent virus belonging to the family *Paramyxoviridae* and also known as Hemagglutinating Virus of Japan (HVJ). The genome of SeV consists of minus single-stranded RNAs (complementary to mRNA) and codes for the six genes of nucleocapsid (NP), polymerase (P), matrix (M), hemagglutinin-neuraminidase (HN), fusion (F), and large (L) proteins. Using HN, SeV adsorbs on sialic acid in the host cell and degrades the same, thereby binding to the cell and introducing their genome into the cytoplasm. The viral genome now within the cell performs gene transcription and replication through the action of L protein it encodes. Unlike with ordinary viruses, the above-described step is performed in the cytoplasm and there is no transfer of the viral genome into the nucleus.

The SeV vector can be prepared as a non-transmissible vector by deleting one or more of F, HN and M genes other than N, P and L genes which are necessary for autonomous replication of the vector within the transfected cell. To prepare an F gene deficient vector, a plasmid having a recombinant vector cDNA inserted downstream of a T7 promoter that is recognized by a bacteriophage T7 derived RNA polymerase, a plasmid that expresses N, P, F and L under the control of the T7 promoter, and a recombinant vaccinia virus that expresses a T7 RNA polymerase are introduced into a monkey kidney derived LLC-MK2 cell. Recently, it has become possible to prepare the desired vector by a convenient method that solely involves transfection with 6 plasmids (a plasmid having a recombinant vector cDNA inserted downstream of a T7 promote, and five plasmids that express N, P, L, F and T7, respectively, under the control of a CAG promoter).

As for reviews about adenoviral vectors and other viral vectors, as well as methods of preparing and using them, skilled artisans may refer to a plurality of reference books. Examples include Gene Therapy Protocols: Methods in Molecular Medicine (ed. by Robbins, Humana Press, 1997), Gene Transfer in Cardiovascular System: Experimental Approaches & Therapeutic Implications (ed. by March, Kluwer Academic Publishers, 1997), and Adenoviral Vectors for Gene Therapy (ed. by Curiel & Douglas, Academic Press, 2002). Kits for preparing adenoviral vectors are also on the commercial market (for example, from Invitrogen in the name of ViraPower™ adenovirus expressing system (#K4930) and from TAKARA BIO in the name of Adenovirus Expression Vector Kit (#6170)) and are readily available for use in carrying out the present invention.

Kits for preparing AAV vectors and SeV vector are also on the commercial market (as AAV Helper-Free system (Stratagene; #240071), the recombinant Sendai virus minivector preparation kit (MBL; DV-0001), etc.) and are readily available for use in carrying out the present invention.

Non-viral expression vectors include but are not limited to vectors that have virus-derived promoters, such as SV (simian virus) 40 virus promoter, cytomegalovirus (CMV) promoter, and Rouse sarcoma virus promoter, and vectors containing a β-actin promoter, or an EF (elongation factor) 1α promoter, and a CAG promoters of hybrid construct consisting of a CMV enhancer and a poly-A-additional signal sequence in the rabbit β-globin gene fused to the avian β-actin promoters (Niwa et al., Gene, 108:193-199, (1991)), and a U6 or H1 promoter which is a Pol. III promoter. Expression vectors that can use these promoters are on the commercial market (for example, from Ambion, Invitrogen, and TAKARA BIO) and readily available

For transfer of the above-mentioned genes or gene vectors, all methods known in the art can be employed and they include: transfection using calcium phosphate or electrical pulses; transfecting into a cell with a liposome or other vehicle that has the gene of interest encapsulated in it; and incorporating the gene of interest into a viral vector such as a retroviral vector or an adenoviral vector. Here, the viral vector means such a construct that the entire or partial length of the nucleic acid sequence of a viral DNA or RNA is deleted or mutated and has the gene of interest incorporated into it in an expressible way.

Specific examples of liposomes that can be used are lipid preparations containing N-[2,3-(dioleyloxy)propyl]-N,N,N-trimethylammonium chloride (DOTMA), dioleoylphosphatidylethanolamine (DOPE), and the like.

### Obtaining specific miRNAs

The present inventors searched through a library of mature miRNAs to identify candidates that would show a marked BrdU uptake ability when they were introduced into cardiomyocytes; as a result, they found that miR-148a (SEQ ID NO: 3), miR-148b (SEQ ID NO: 5), miR-152 (SEQ ID NO: 7) and miR-373 (SEQ ID NO: 9) were mature miRNAs having the action for promoting the proliferation of cardiomyocytes, and they used these mature miRNAs in further experiments.

Precursors of these mature miRNAs are also included as preferred miRNAs that can be used in the present invention. Exemplary precursors of mature miRNAs include primary miRNA transcripts (pri-miRNA) or precursor miRNAs (pre-miRNA) of the respective miRNAs. Specific examples of the preferred miRNAs that can be used in the present invention include the following which are primary transcripts (pri-miRNA) of the genes coding for the specific mature miRNAs that have been mentioned above: mir-148a (precursor of miR-148a, SEQ ID NO: 4), mir-148b (precursor of miR-148b, SEQ ID NO: 6), mir-152 (precursor of miR-152, SEQ ID NO: 8), and mir-373 (precursor of miR-373, SEQ ID NO: 10).

[Table 1]

**Table 1:**

| Name of miRNA | Sequence | SEQ ID NO |
|---|---|---|
| miR-148a | ucagugcacu acagaacuuu gu | 3 |
| mir-148a | gaggcaaagu ucugagacac uccgacucug aguaugauag aagucagugc acuacagaac uuugucuc | 4 |
| miR-148b | ucagugcauc acagaacuuu gu | 5 |
| mir-148b | caagcacgau uagcauuuga ggugaaguuc uguuauacac ucaggcugug gcucucugaa agucagugca ucacagaacu uugucucgaa agcuuucua | 6 |
| miR-152 | ucagugcaug acagaacuug g | 7 |
| mir-152 | uguccccccc ggcccagguu cugugauaca cuccgacucg ggcucuggag cagucagugc augacagaac uugggcccgg aaggacc | 8 |
| miR-373 | gaagugcuuc gauuuugggg ugu | 9 |
| mir-373 | gggauacuca aaaugggggc gcuuuccuuu uugucuguac ugggaagugc uucgauuuug ggguguccc | 10 |

The miRNAs that can be used in the present invention encompass variants and analogs of the mature miRNAs and precursor miRNAs that are identified in Table 1 above, as well as precursors of miRNAs other than the precursors of miRNAs that are identified in Table 1 above (as exemplified by primary miRNA transcripts (pri-miRNA)), as well as variants and analogs thereof.

For example, miR-148a (SEQ ID NO: 3), miR-148b (SEQ ID NO: 5) and miR-152 (SEQ ID NO: 7) which can be used in the present invention have the seed sequence ucagugca (SEQ ID NO: 1) in common on the 5' side of the respective miRNA to make up a miR-148 family. The sequences of the respective miRNAs that belong to the miR-148 family are indicated below in alignment, with the conserved seed sequences underlined.
miR-148a: ucagugcacuacagaacuuugu (SEQ ID NO: 3)
miR-148b: ucagugcaucacagaacuuugu (SEQ ID NO: 5)
miR-152: ucagugcaugacagaacuugg (SEQ ID NO: 7).

Hence, a group of miRNAs according to the present invention comprises mature miRNAs that contain the seed sequence of ucagugca (SEQ ID NO: 1) at the 5' end and precursors of said miRNAs, as well as variants or analogs thereof. These miRNAs comprise miRNAs selected from the group consisting of miR-148a (SEQ ID NO: 3), miR-148b (SEQ ID NO: 5), miR-152 (SEQ ID NO: 7), precursors of said miRNAs, as well as variants and analogs thereof. For example, precursors of mature miRNAs, i.e., miR-148a (SEQ ID NO: 3), miR-148b (SEQ ID NO: 5) and miR-152 (SEQ ID NO: 7), include pri-miRNAs and pre-miRNAs of the respective miRNAs. Specific examples include precursors represented by SEQ ID NOS: 4, 6 and 8 depicted below.

human mir-148a: gaggcaaagu ucugagacac uccgacucug aguaugauag aagucagugc acuacagaac uuugucuc (SEQ ID NO: 4)
human mir-148b: caagcacgau uagcauuuga ggugaaguuc uguuauacac ucaggcugug gcucucugaa agucagugca ucacagaacu uugucucgaa agcuuucua (SEQ ID NO: 6)
human mir-152: uguccccccc ggcccagguu cugugauaca cuccgacucg ggcucuggag cagucagugc augacagaac uugggcccgg aaggacc (SEQ ID NO: 8).

Another type of mature miRNA, or miR-373, which can be used in the present invention has the common seed sequence gaagugcu (SEQ ID NO: 2) on the 5' side of the miRNA to be a member of a miR-373 family. The sequence of the miRNA in the miR-373 family is indicated below, with the seed sequence underlined.

miR-373: gaagugcuucgauuuuggggugu (SEQ ID NO: 9).

Hence, another group of miRNAs according to the present invention comprises mature miRNAs that contain the seed sequence of gaagugcu (SEQ ID NO: 2) at the 5' end and precursors of said miRNAs, as well as variants or analogs thereof. These miRNAs comprise miRNAs selected from the group consisting of miR-373 (SEQ ID NO: 9), precursors of said miRNA, as well as variants and analogs thereof. For example, precursors of the mature miRNA, i.e., miR-373 (SEQ ID NO: 9), include pri-miRNAs and pre-miRNAs of this miRNA. Specific examples include the precursor represented by SEQ ID NO: 10 depicted below.

human miR-373: gggauacuca aaaugggggc gcuuuccuuu uugucuguac ugggaagugc uucgauuuug ggguguccc (SEQ ID NO: 10).

The thus obtained miRNAs according to the present invention were found to show a marked activity for proliferating cardiomyocytes.

In contrast, the miRNAs according to the present invention did not show any significant activity for promoting the proliferation of established normal cell lines, such as MRC-5 (derived from human embryonic lung), WI-38 (derived from human embryonic lung), and MC3T3-E1 (derived from mouse scull cap).

### Application in gene therapy

In another embodiment, the present invention provides pharmaceutical compositions for use in gene therapy to carry out the invention, which contain expression vectors such as viral or non-viral vectors that contain nucleic acids that encode miRNAs having an action for promoting the proliferation of cardiomyocytes (said vectors are hereinafter referred to simply as "gene expression vectors"), preferably containing viral vectors, more preferably containing adenoviral vectors or HVJ vector, AAV vectors, lentiviral vectors, etc. The miRNA defining nucleic acids to be contained in the gene expression vectors may be of any of the miRNA defining nucleic acids that have been described in the section "Obtaining specific miRNAs." Since the pharmaceutical compositions for use in gene therapy contain nucleic acids that encode miRNAs having an action for promoting the proliferation of cardiomyocytes, they can be used as myocardial regenerators or therapeutics for heart disease on account of the actions of miRNAs. The heart disease that can be treated is not particularly limited if it involves the weakening, disabling or dying of cardiomyocytes; specific examples include myocardial infarction, ischemic cardiac disease, congestive heart failure, hypertrophic cardiomyopathy, dilated cardiomyopathy, myocarditis, chronic heart failure, etc.

The dosage forms of the pharmaceutical compositions are not particularly limited and conventional methods can be used to formulate pharmaceutical preparations. For example, they may be in the form of injectable preparations that contain the gene expression vectors of the present invention in pharmaceutically acceptable drug carriers or dilutions such as sterilized water or buffered physiological saline. Drug carriers may additionally contain suitable stabilizers (e.g. nuclease inhibitor), chelating agents (e.g. EDTA) and/or other auxiliary agents. The pharmaceutical compositions containing those ingredients may optionally be filtered or otherwise sterilized and then charged into containers such as aseptic ampoules.

The pharmaceutical compositions of the present invention are administered in doses that need be appropriately adjusted depending on various conditions such as the age, sex, body weight and symptoms of the patient, as well as the route of administration, and anyone skilled in the art can appropriately set the required dose. Generally, the dose is within the range of from about 1.0 µg/kg to about 1.0 g/kg, preferably from about 10 µg/kg to about 100 mg/kg, daily per adult in terms of the DNA content of the active ingredient. If a viral vector such as an adenoviral vector is to be used, the final titer of the virus is preferably from 10⁷ to 10¹³ pfu/mL, more preferably from 10⁹ to 10¹² pfu/mL.

Particularly in the case where the gene expression vector contained in the pharmaceutical compositions of the present invention is based on a non-viral vector, it may be supplied as a complex with a liposome. This form of vector has a potential to realize high transfection efficiency, especially in cardiomyocytes. A lot of new lipid preparations containing N-[2,3-(diolecyloxy)propyl]-N,N,N-trimethylammonium chloride (DOTMA), dioleoylphosphaditylethanolamine (DOPE) or the like have been developed as specific examples of liposome and various cell lines have been tested for transfection (Banerjee, Journal of Biomaterials Applications, 16:3-21, (2001); Maurer et al., Expert Opinion on Biological Therapy, 1:923-947, (2001)). Also effective is the so-called HVJ-liposome method which uses a fusion forming liposome containing an HVJ-derived envelope (Yonemitsu et al., International Journal of Oncology, 12:1277-1285, (1998); Kaneda et al., Molecular Medicine Today, 5:298-303, (1999)). These liposomes which are intended for nucleic acid transfer are on the commercial market and Lipofectamine RNAi MAX (Invitrogen), Oligofectamine (Invitrogen), siLentFect (BIO-RAD), HiPerFect (Qiagen) and the like can be used as liposomes that are particularly intended for miRNA transfer.

The above-described gene expression vectors or pharmaceutical compositions that contain them may be introduced into the whole heart of a patient with heart disease but the introduction is preferably localized at the diseased site. The term "diseased site" as referred to in the present invention means a site where cardiomyocytes in an individual (human or non-human animal, as applicable hereinafter) are weakened, disabled or dead as well as a region nearby, or a site where cardiomyocytes in an individual are likely to be weakened or disabled progressively or to die. In the case under consideration, the way to introduce the gene expression vectors or pharmaceutical compositions that contain them into the diseased site may be by transferring the preparation continuously to the diseased site using an osmotic pump, an osmotic tube or the like. Other applicable methods include direct injection into the heart with a syringe after thoracotomy and transvascular (indirect) injection through a catheter under radioscopy.

The transvascular approach is preferred since gene transfer can be localized at the heart and, in this case, the gene expression vectors or pharmaceutical compositions that contain them can be injected into a blood vessel for transfer to cardiomyocytes via the blood stream or, alternatively, they may be directly injected into the myocardium to contact cardiomyocytes. Such techniques of surgical operation using a catheter or the like are known in the art concerned and books that can be referred to include standard textbooks such as Gene Transfer in Cardiovascular System: Experimental Approaches & Therapeutic Implications (ed. by March, Kluwer Academic Publishers, 1997), Vascular Surgery, 5th ed. (Rutherford, W.B. Saunders, 2000), and Textbook of Interventional Cardiology, 4th ed. (ed. by Topol, W.B. Saunders, 2002). Catheters that may be used to implement the above-described methods are on the commercial market (for example, from Boston Scientific and Edwards Lifesciences Corporation) and readily available.

### Use of cardiomyocytes proliferated by the method of the present invention

The cardiomyocytes proliferated by the method according to the present invention are subsequently harvested, isolated and purified by known methods to achieve efficient and high-yield production of high-purity cardiomyocytes. The thus obtained cardiomyocytes are hereinafter referred to as cardiomyocytes prepared by the present invention.

To purify cardiomyocytes, any known methods for cell isolation and purification may be employed and specific examples include antigen-antibody reaction based methods such as flow cytometry, use of magnetic beads or panning, as well as cell fractionation that involves density gradient centrifugation using a carrier such as sucrose or Percoll.

Another cardiomyocyte sorting method that can be used is such that a gene for a cardiomyocyte source, such as an animal cell or a stem cell like an ES cell, is preliminarily modified to be provided with drug resistance or the ability to express an ectopic protein and cardiomyocytes are selectively harvested with reference to these markers. For example, Field and his coworkers reported the following: a gene cassette capable of expressing a neomycin (G418) resistance gene under the control of an α-type myosin heavy-chain promoter was introduced into a mouse ES cell to construct a system in which the ES cell would differentiate into a cardiomyocyte which could survive in a G418-containing medium only when the α-type myosin heavy-chain promoter was consequently expressed; the cells sorted as G418 resistant cells were found to be cardiomyocytes with a probability of 99% or higher (USP 6,015,671; Klug et al., The Journal of Clinical Investigation, 98:216-224, (1996)).

In yet another embodiment, the cardiomyocytes prepared by the present invention are useful in assaying the pharmacological properties and activities of various physiologically active substances (for example, drugs), novel gene products with unknown functions, and the like. For example, they can be used in screening to look for substances or drugs that are associated with functional adjustments of cardiomyocytes, as well as substances or drugs that are toxic or harmful to cardiomyocytes. In a still another embodiment, an assay kit containing the cardiomyocytes prepared by the present invention is useful for the screening process described above.

The test substance that may be subjected to the screening process is of any kind that can be added to a culture system and may be exemplified by low-molecular weight compounds, high-molecular weight compounds, organic compounds, inorganic compounds, proteins, peptides, genes, viruses, cells, cell culture supernatants, and microbial cultures. Efficient gene transfer into culture systems can be performed by various methods, such as adding a gene to the culture system using a viral vector such as a retroviral or adenoviral vector or adding the gene as it is encapsulated in a liposome or the like.

The test substance can be evaluated by measuring qualitative or quantitative changes in the functions of cardiomyocytes. To take the viability as an example, the cardiomyocytes prepared by the present invention are sown on a culture plate to give an appropriate cell density and cultured in a serum-free medium to induce cell death (apoptosis) and in the process, a suitable amount of the test substance may be added to the medium to measure the survival rate or mortality of cardiomyocytes. The viability or mortality of cardiomyocytes may be measured by visual observation with the uptake of dye (e.g. trypan blue) being used as a marker, or by a method using a dehydrogenase activity (reducing ability) as a marker, or even by a method using the caspase activity or the expression of annexin V as a marker, both being specific for apoptotic cells. Kits depending upon said mechanism are on the commercial market (for example, from Sigma, Clonetech, and Promega) and readily available.

The substances and drugs obtained by the screening methods described above have the action for inducing the differentiation into cardiomyocytes or adjusting the functions of cardiomyocytes and, hence, can be used as drugs for preventing or treating heat diseases including myocardial infarction, ischemic cardiac disease, congestive heart failure, hypertrophic cardiomyopathy, dilated cardiomyopathy, myocarditis, and chronic heart failure. These compounds may be novel or known compounds.

The cardiomyocytes prepared by the present invention can also be used as grafting cells in myocardial regeneration or treatment of heart disease. Heart diseases that can be treated may include myocardial infarction, ischemic cardiac disease, congestive heart failure, hypertrophic cardiomyopathy, dilated cardiomyopathy, myocarditis, and chronic heart failure. Grafting cells that can be used may take on any forms as long as the cardiomyocytes prepared by the present invention are contained at high purity and they may include a suspension of the cells in an aqueous vehicle such as a medium, an embedment of the cells in a solid-phase carrier such as a biodegradable substrate, or the cardiomyocytes that have been processed into a mono- or multi-layered cell sheet (Shimizu et al., Circulation Research, 90:e40-e48, (2002)).

Methods for transplanting the above-described grafting cardiomyocytes to the diseased site include, but are not limited to, direct injection into the heart with a syringe after thoracotomy, implantation after part of the heart is surgically incised, and even implantation by a transvascular method using a catheter (Murry et al., Cold Spring Harbor Symposia on Quantitative Biology, 67:519-526, (2002); Menasche, The Annals of Thoracic Surgery, 75:S20-S28, (2003); Dowell et al., Cardiovascular Research, 58:336-350, (2003)). It has been reported that extremely good therapeutic effects were obtained when cardiomyocytes recovered from an embryonic heart were implanted in the heart of an animal with cardiac disorder by those methods (Menasche, The Annals of Thoracic Surgery, 75:S20-S28, (2003); Reffelmann et al., Heart Failure Reviews, 8:201-211, (2003)). Cardiomyocytes derived from ES cells exhibit phenotypes quite similar to those of cardiomyocytes derived from an embryonic heart (Maltsev et al., Mechanisms of Development, 44:41-50, (1993); Maltsev et al., Circulation Research, 75:233-244, (1994)). In an animal experiment involving actual implantation of ES-cell derived cardiomyocytes into an adult heart, the cell viability was confirmed to be extremely high and almost comparable to the levels obtained in the case of grafting embryonic cardiac muscle cells (Klug et al., The Journal of Clinical Investigation, 98:216-224, (1996)). Thus, it can be expected that in the above-mentioned heart diseases due to the exhaustion and loss of cardiomyocytes, the cardiomyocytes prepared by the method described hereinabove will help to improve cardiac functions if they are transplanted to replace the diseased heart tissue.

### EXAMPLES

On the following pages, the present invention is described more specifically by reference to examples, which are given here for illustrative purposes only and are by no means intended to limit the scope of the present invention.

### Example 1: BrdU-Uptake-Based Screening of miRNA Library Using Primary Culture of Cardiomyocytes

The heart was excised from 2-4 day old rats (Wistar rats obtained from SHIMIZU Laboratory Supplies Co., Ltd.) and treated with collagenase to prepare a cell population, from which cardiomyocyte fractions were recovered by Percoll density gradient centrifugation (a Percoll suspension of cells with a density of 1.082 g/mL was layered on a Percoll solution with a density of 1.050 g/mL or 1.060 g/mL, and subjected to centrifugation at 4 °C and 3000 rpm (2000 G) for 25 minutes). The thus obtained cardiomyocytes were suspended in an Eagle minimum medium (nakalai tesque) supplemented with 5% fetal calf serum (FCS; SAFC Bioscience) and then seeded on culture dishes for cell culture in a CO₂ incubator at 37 °C. The thus prepared cardiomyocytes (15,000 cells/well on a 96-well plate) were transfected with 2 pmol each of Pre-miR™ miRNA Precursor Library-HumanV2 (Ambion : a library designed to mimic the 328 human mature miRNAs listed in miRBase Sequence Database Version 8.0) using Lipofectamine™ RNAiMAX (Invitrogen) and 2 days later, the BrdU uptake was measured using a cell proliferation ELISA, BrdU chemiluminescence kit (Roche) and compared with the BrdU uptake by a negative control or the group to which Pre-miR™ miRNA Precursor Molecule-Negative control #1 (Ambion) had been added; the miRNAs that showed a markedly high BrdU uptake effect were selected as candidate miRNAs that would markedly promote the DNA synthesis ability. In the test described above, the thus selected candidate miRNAs were miR-148a, miR-148b, miR-152, and miR-373.

[Table 2]

**Table 2: BrdU uptake of mature miRNA**

| miRNA | SEQ ID NO | BrdU value |
|---|---|---|
| miR-148a | 3 | 2.7 |
| miR-148b | 5 | 2.3 |
| miR-152 | 7 | 2.6 |
| miR-373 | 9 | 2.9 |

### Example 2: Analysis of a Marker for the Progress of Cell Cycle by Forced miRNA Expression in Primary Culture of Cardiomyocytes

Cardiomyocytes (100,000 cells/well on a 24-well plate) were transfected with 1 pmol of Pre-miR™ miRNA Precursor Molecules (Ambion) that were designed to mimic miR-148a, miR-148b, miR-152 and miR-373 (the candidate mature miRNAs obtained in Example 1) and 1 pmol of Negative control #1 (a negative control, also available from Ambion) using Lipofectamine™ RNAiMAX (Invitrogen) and 2-4 days after the transfection, the cells were fixed with 4% paraformaldehyde.

Following subsequent reaction with anti-Ki67 antibody (Thermo SCIENTIFIC) (1:200 dilution) and anti-Troponin T antibody (Thermo SCIENTIFIC) (1:200 dilution), the cells were stained with Alexa Fluor™ labeled antibody (Alexa-488 or Alexa-568; Molecular Probes) (both 1:400 dilutions). The cell nucleus was stained with a 4,6-diamidine-2'-phenylindole diydrochloride: DAPI) solution (1µg/mL).

The cardiomyocytes immunostained by the method described above were analyzed with In Cell Analyzer1000 (GE Healthcare) to measure their Ki67 positive rate. The Ki67 nucleoprotein is of such a nature that it is expressed in cells proliferating at all stages of the cell cycle (Scholzen & Gerdes, Journal of Cellular Physiology, 182:311-22, 2000), so it was used as a marker to detect cells that were in the process of continued cell cycle.

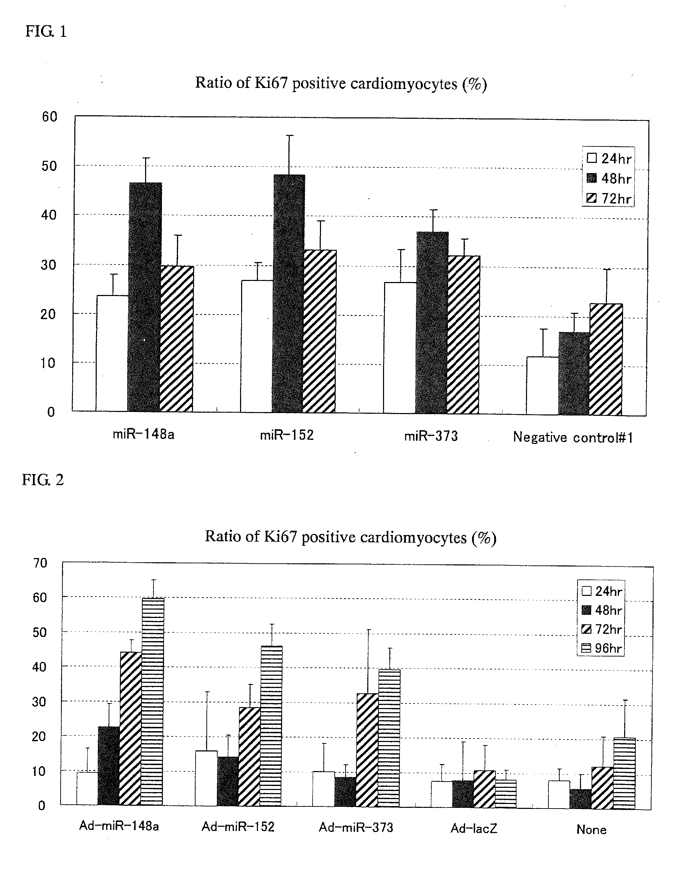

The results are shown in FIG. 1.

In view of these results, miR-148a, miR-148b, miR-152 and miR-373 were identified as miRNAs having a marked action for promoting the proliferation of cardiomyocytes in comparison with the case of using Negative control #1.

### Example 3: Analysis of a Marker for the Progress of Cell Cycle by Adenovirus-Forced miRNA Expression in Primary Culture of Cardiomyocytes

Adenoviral vector for expressing miR-148a, miR-152, and miR-373 were prepared by using ViraPower™ Adenoviral Expression System (Invitrogen). To be more specific, human genomic DNA fragments comprising the precursor sequences of the respective miRNAs and adjacent several hundred nucleotides were amplified by PCR using the primers shown below, with the template being human genomic DNAs.
hsa-miR-148a-F : 5'-caccgaacacacctgcaggaagaaact-3' (SEQ ID NO: 11)
hsa-miR-148a-R : 5'-gttcccatttacagggtttaaccca-3' (SEQ ID NO: 12)
hsa-miR-152-F : 5'-caccgtcccagactcggctcccatca-3' (SEQ ID NO: 13)
hsa-miR-152-R : 5'-actcgaggtggacaccctgtgt-3' (SEQ ID NO: 14)
hsa-miR-373-F : 5'-caccgtgaccaaggggctgtatgca-3' (SEQ ID NO: 15)
hsa-miR-373-R : 5'-ctgcccaccccagaatatgcca-3' (SEQ ID NO: 16).

The resulting PCR products were inserted into a pENTR/D-TOPO vector (Invitrogen) and after checking their base sequences, recombination with a pAd/CMV/V5-DEST vector (Invitrogen) was performed with a Gateway system (Invitrogen) to prepare pAd/CMV-miR-148a, pAd/CMV-miR-152, and pAd/CMV-miR-373. Subsequently, these vectors were digested with the restriction enzyme PacI and transfected into human embryonic kidney derived 293A cells using Lipofectamine2000 (Invitrogen) to thereby prepare recombinant adenoviruses Ad-miR-148a, Ad-miR-152, and Ad-miR-373. The recombinant adenoviruses prepared by the above-described method were so constructed as to express the respective miRNA precursor inserts under the control of the CMV promoter, so they would be capable of high-yield expression within mammalian cells. The miRNA precursors expressed in the mammal are processed by a group of enzymes within the mammalian cells to become functional mature miRNAs.

Also used in the above-described experiment was Ad-LacZ (TAKARA BIO) which is a commercial recombinant adenovirus for expressing LacZ.

Subsequently, a high-titer viral solution was prepared for each of the recombinant viruses and the titers of the prepared viral solutions as determined by a plaque assay using 293A cells were all within the range of 10⁹ - 10¹⁰ pfu/mL. Hereinafter, the number of live virus particles with which one cell is infected is designated as the multiplicity of infection (moi); hence, infecting one cell with one virus particle is indicated by moi = 1.

In addition, the prepared recombinant viruses were added to cardiomyocytes at moi = 10-1000 and 2 days later, miRNA-containing total RNAs were recovered using a miRNeasy mini kit (Qiagen). From 10 ng of the recovered total RNAs, cDNAs were synthesized using the reverse transcription primers specific for the respective miRNAs that were accessories to a TaqMan MicroRNA reverse transcription kit (Applied Biosystems) and a TaqMan MicroRNA Assay (Applied Biosystems) and, subsequently, using the forward and reverse primers and TaqMan probes that were specific for the respective miRNAs and which were accessories to the TaqMan MicroRNA Assay (Applied Biosystems), real-time PCR was performed with an Applied Biosystems 7900HT Fast Real time PCR system and the expressions of the mature miRNAs were quantified.

Using RNU6B as an endogenous control, the amounts of expression were normalized. As a result, it was confirmed that the mature miRNAs were expressed in a viral dose dependent manner.

The thus prepared recombinant viruses Ad-miR-148a, Ad-miR-152 and Ad-miR-373 as well as the negative control Ad-LacZ were added to cardiomyocytes (100,000 cells/well on a 24-well plate) at moi = 10-100 and 2-4 days later, the cells were fixed with 4% paraformaldehyde.

Following subsequent reaction with anti-Ki67 antibody (Thermo SCIENTIFIC) (1:200 dilution) and anti-Troponin T antibody (Thermo SCIENTIFIC) (1:200 dilution), the cells were stained with Alexa Fluor™ labeled antibody (Alexa-488 or Alexa-568; Molecular Probes) (both 1:400 dilutions). The cell nucleus was stained with a DAPI solution (1µg/mL).

The cardiomyocytes immunostained by the method described above were analyzed with In Cell Analyzer1000 to measure their Ki67 positive rate.

The results are shown in FIG. 2, in which "None" refers to the untreated cells.

In view of these results, each of miR-148a, miR-152 and miR-373 was positive for the cell cycle progression marker Ki67 in contrast with the negative control (LacZ), indicating an enhanced cell cycle progression of the cardiomyocytes; it was thus verified that those miRNAs had an action for promoting the proliferation of cardiomyocytes.

### Example 4: Analysis of a Marker for Cell Division by Forced miRNA Expression or Adenovirus-Forced miRNA Expression in Primary Culture of Cardiomyocytes

Cardiomyocytes were transfected with miR-148a, miR-148b, miR-152, miR-373 and Negative control #1 (a negative control) by the same procedure as in Example 2. In another experiment, Ad-miR-148a, Ad-miR-152, Ad-miR-373 and the negative control Ad-LacZ were added to cardiomyocytes by the same procedure as in Example 3. In each experiment, the cells were fixed with 4% paraformaldehyde 2 or 3 days after the transfection or addition.

Following subsequent reaction with anti-phosphorylated histone H3 (H3P) antibody (MILLIPORE) (1:200 dilution) and anti-Troponin antibody (Thermo SCIENTIFIC) (1:200 dilution), the cells were stained with Alexa Fluor™ labeled antibody (Alexa-488 or Alexa-568; Molecular Probes) (both 1:400 dilutions). The cell nucleus was stained with a DAPI solution (1µg/mL).

The cardiomyocytes immunostained by the method described above were analyzed with In Cell Analyzer1000 to measure their H3P positive rate. The phosphorylation of Ser-10 in histone H3 is closely related to chromatin condensation that occurs during mitotic cell division (Adamas R, The Journal of Cell Biology, 153: p. 865-880, 2001), so it was used as a marker for cell division.

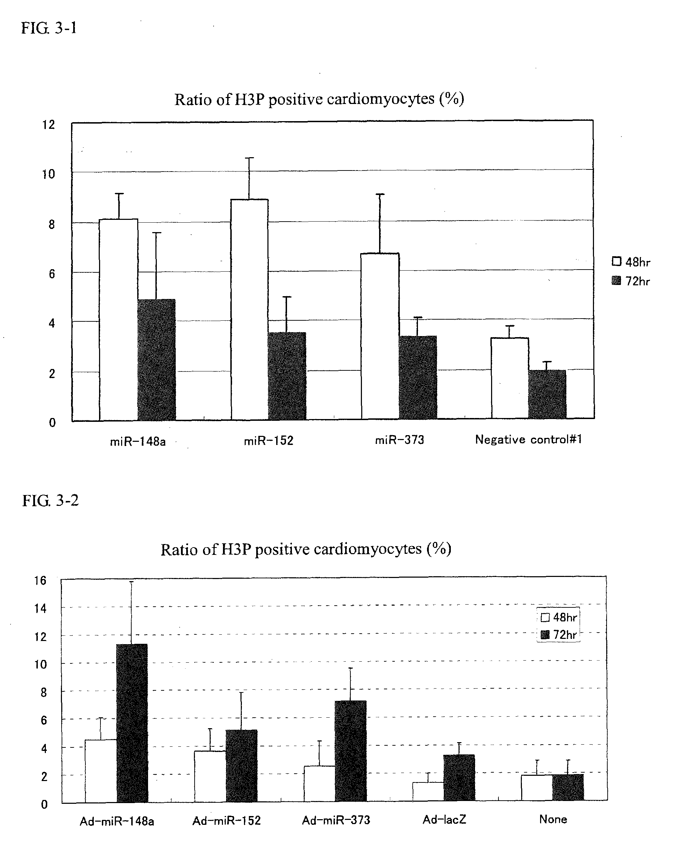

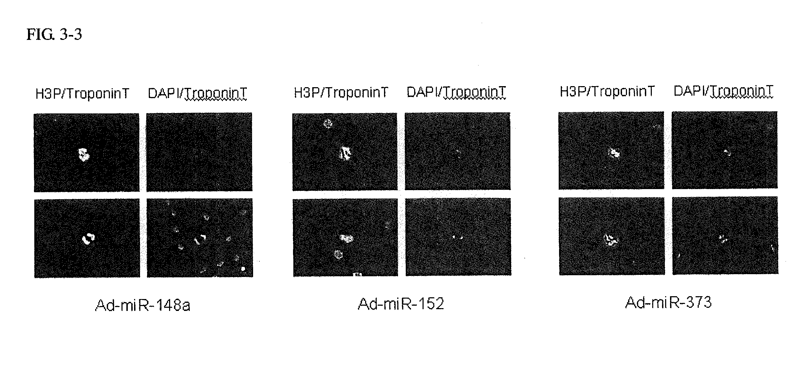

The results are shown in FIGs. 3-1 and 3-2, in which "None" refers to the untreated cells. Micrographs of the immunostained cells are shown in FIG. 3-3.

As these results show, each of miR-148a, miR-152 and miR-373 was positive for the cell division marker H3P in contrast with the two negative controls, Negative control #1 (in the case of FIG. 3-1) and LacZ (in the case of FIG. 3-2), and cells at mitotic division and cytoplasmic division stages were observed; hence, the cell division of cardiomyocytes was enhanced, verifying that those microRNAs had an action for promoting the proliferation of cardiomyocytes.

### Example 5: Study for the Possibility of Cardiomyocytes to Proliferate In Vivo

To see whether the forced expression of miR-148a, miR-152 or miR-373 would trigger proliferation of cardiomyocytes *in vivo,* Wistar rats (250-300 g) were opened in the chest and under visual examination, the recombinant viruses Ad-miR-148a, Ad-miR-152 and Ad-miR-373 (each being 1×10⁹pfu/mL) that were prepared in Example 3 were each injected in a total amount of 200 µL into 4 sites of the apical region by means of a 30 G needle. As a negative control, miR-141 was used and the sequence of interest was amplified by PCR in accordance with the procedure of Example 3 using the primers shown below, with the template being human genomic DNAs:
hsa-miR-141-F : 5'-caccgcagggatcctgggcctga-3' (SEQ ID NO: 17)
hsa-miR-141-R : 5'-cgggaagacaatggaggtgcct-3' (SEQ ID NO: 18).

The resulting PCR product was processed as in Example 3 to prepare Ad-miR-141 and under visual examination, Ad-miR-141 (1×10⁹pfu/mL) was injected into 4 sites of the apical region of Wistar rats (250-300 g) under thoracotomy in a total amount of 200 µL through a 30 G needle.

Four days after the injection, 4% paraformaldehyde was perfused to fix the heart tissue and sections were prepared; following subsequent reaction with anti-Ki67 antibody (Thermo SCIENTIFIC) (1:500 dilution) and anti-Troponin T antibody (Thermo SCIENTIFIC) (1:600 dilution), the sections were stained with Alexa Fluor™ labeled antibody (Alexa-488 or Alexa-568; Molecular Probes) (both 1:400 dilutions) so that they were visible in green or red. The cell nucleus was stained with a DAPI solution (1µg/mL) so that it was visible in blue.

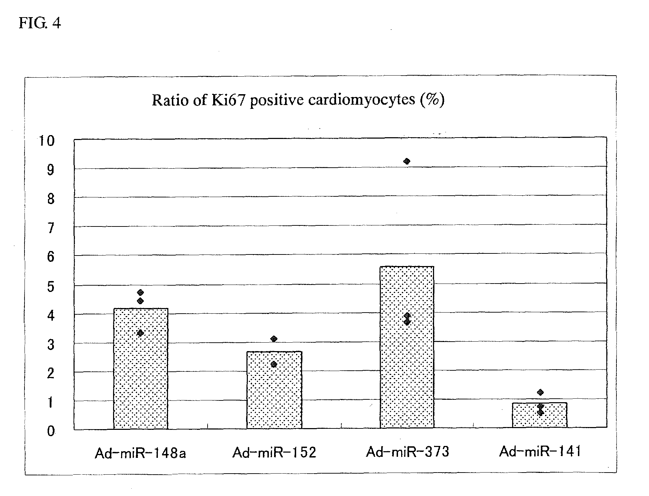

The stained heart tissue sections were examined under a fluorescent microscope and the relative proportions of cardiomyocytes (the cells positive for both DAPI and Troponin T) and cardiomyocytes in the process of continued cell cycle (the cells positive for both Ki67 and Troponin T) were measured. The number of cells to be measured in each individual was at least 300. The results are shown in FIG. 4, from which it is obvious that the proportion of Ki67-positive cardiomyocytes increased in the hearts transfected with the adenovirus of miR-148a, miR-152 or miR-373. In contrast, Ki67-positive cardiomyocytes were hardly found in the hearts transfected with the adenovirus of the negative control miR-141.

As these results show, the cell cycle progression marker Ki67 became positive upon *in vivo* expression of miR-148a, miR-152 or miR-373, indicating that the cell cycle of cardiomyocytes was enhanced to have them acquire the ability to proliferate.

### SEQUENCE LISTING FREE TEXT

SEQ ID NO: 1: seed sequence common to the 5' side of a miR-148 family;
SEQ ID NO: 2: seed sequence common to the 5' side of a miR-373 family;
SEQ ID NO: 11: forward primer, hsa-miR-148a-F, for amplifying the coding region of miR-148a gene;
SEQ ID NO: 12: reverse primer, hsa-miR-148a-R, for amplifying the coding region of miR-148a gene;
SEQ ID NO: 13: forward primer, hsa-miR-152-F, for amplifying the coding region of miR-152 gene;
SEQ ID NO: 14: reverse primer, hsa-miR-152-R, for amplifying the coding region of miR-152 gene;
SEQ ID NO: 15: forward primer, hsa-miR-373-F, for amplifying the coding region of miR-373 gene;
SEQ ID NO: 16: reverse primer, hsa-miR-373-R, for amplifying the coding region of miR-373 gene;
SEQ ID NO: 17: forward primer, hsa-miR-141-F, for amplifying the coding region of miR-141 gene;
SEQ ID NO: 18: reverse primer, hsa-miR-141-R, for amplifying the coding region of miR-141 gene;

For other sequences, see Table 1.

Furthermore, the present invention relates to the following items:
1. A pharmaceutical composition for the treatment of heart disease comprising a nucleic acid that encodes a miRNA having a cardiomyocyte proliferation promoting action.
2. The pharmaceutical composition as recited in item 1, wherein the miRNA is a substance selected from the group consisting of a mature miRNA, a precursor of said miRNA, as well as variants and analogs thereof.
3. The pharmaceutical composition as recited in item 1 or 2, wherein the miRNA is a substance comprising a seed sequence depicted by SEQ ID NO: 1 or SEQ ID NO: 2.
4. The pharmaceutical composition as recited in item 3, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof.
5. The pharmaceutical composition as recited in item 3, wherein the substance comprising the seed sequence depicted by SEQ ID NO: 2 is a substance selected from the group consisting of miR-373 (SEQ ID NO: 9), precursors thereof, as well as variants and analogs thereof.
6. The pharmaceutical composition as recited in any one of items 1 to 5, wherein the nucleic acid that encodes the miRNA having a cardiomyocyte proliferation promoting action is contained as an expression vector for introduction and expression in cardiomyocytes.
7. The pharmaceutical composition as recited in item 6, wherein the vector is a viral vector.
8. The pharmaceutical composition as recited in any one of items 1 to 7, wherein the miRNA is a miRNA derivative comprising at least one modified internucleoside linkage, at least one modified sugar moiety, at least one modified base, or any combinations thereof.
9. The pharmaceutical composition as recited in any one of items 1 to 8, wherein the heart disease is myocardial infarction, ischemic cardiac disease, congestive heart failure, hypertrophic cardiomyopathy, dilated cardiomyopathy, myocarditis, or chronic heart failure.
10. A method for the treatment of heart disease wherein a nucleic acid that encodes the miRNA having a cardiomyocyte proliferation promoting action is introduced into a damaged site in the heart of an individual to thereby proliferate cardiomyocytes at said site.
11. Use of a nucleic acid that encodes the miRNA having a cardiomyocyte proliferation promoting action for the manufacture of pharmaceutical compositions for the treatment of heart disease.
12. A nucleic acid that encodes the miRNA having a cardiomyocyte proliferation promoting action for use in the treatment of heart disease.
13. A method for proliferating cardiomyocytes wherein a nucleic acid that encodes the miRNA having a cardiomyocyte proliferation promoting action is introduced and expressed in cardiomyocytes.
14. The method as recited in item 13, wherein the miRNA is a substance selected from the group consisting of a mature miRNA, a precursor of said miRNA, as well as variants and analogs thereof.
15. The method as recited in item 13 or 14, wherein the miRNA is a substance comprising a seed sequence depicted by SEQ ID NO: 1 or SEQ ID NO: 2.
16. The method as recited in item 15, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof.
17. The method as recited in item 15, wherein the substance comprising the seed sequence depicted by SEQ ID NO: 2 is a substance selected from the group consisting of miR-373 (SEQ ID NO: 9), precursors thereof, as well as variants and analogs thereof.
18. The method d as recited in any one of item 13 to 17, wherein the nucleic acid that encodes the miRNA having a cardiomyocyte proliferation promoting action is introduced into cardiomyocytes using an expression vector.
19. The method as recited in item 18, wherein the vector is a viral vector.
20. The method as recited in any one of items 13 to 19, wherein the miRNA is a miRNA derivative comprising at least one modified internucleoside linkage, at least one modified sugar moiety, at least one modified base, or any combinations thereof.

## Claims

1. A pharmaceutical composition for use in the treatment of heart disease comprising a nucleic acid that encodes a miRNA, wherein the miRNA is a substance comprising a seed sequence depicted by SEQ ID NO: 1, wherein the miRNA has a cardiomyocyte proliferation promoting action.

2. The pharmaceutical composition as recited in claim 1, wherein the miRNA is a substance selected from the group consisting of a mature miRNA, a precursor of said miRNA, as well as variants and analogs thereof.

3. The pharmaceutical composition as recited in claim 1, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof.

4. The pharmaceutical composition as recited in any one of claims 1 to 3, wherein the nucleic acid that encodes the miRNA having a cardiomyocyte proliferation promoting action is contained as an expression vector for introduction and expression in cardiomyocytes.

5. The pharmaceutical composition as recited in claim 4, wherein the vector is a viral vector.

6. The pharmaceutical composition as recited in any one of claims 1 to 5, wherein the miRNA is a miRNA derivative comprising at least one modified internucleoside linkage, at least one modified sugar moiety, at least one modified base, or any combinations thereof.

7. The pharmaceutical composition as recited in any one of claims 1 to 6 wherein the heart disease is myocardial infarction, ischemic cardiac disease, congestive heart failure, hypertrophic cardiomyopathy, dilated cardiomyopathy, myocarditis, or chronic heart failure.

8. An in vitro method for proliferating cardiomyocytes wherein a nucleic acid that encodes the miRNA, wherein the miRNA is a substance comprising a seed sequence depicted by SEQ ID NO: 1, wherein the miRNA has a cardiomyocyte proliferation promoting action is introduced and expressed in cardiomyocytes.

9. The method as recited in claim 8, wherein the miRNA is a substance selected from the group consisting of a mature miRNA, a precursor of said miRNA, as well as variants and analogs thereof.

10. The method as recited in claim 8 or 9, wherein the substance comprising the seed sequence depicted by SEQ ID NO: 1 is a substance selected from the group consisting of miR-148a (SEQ ID NO: 3), miR-148b (SEQ ID NO: 5), miR-152 (SEQ ID NO: 7), precursors thereof, as well as variants and analogs thereof.

11. The method as recited in any one of claim 8 to 10, wherein the nucleic acid that encodes the miRNA having a cardiomyocyte proliferation promoting action is introduced into cardiomyocytes using an expression vector.

12. The method as recited in claim 11, wherein the vector is a viral vector.

13. The method as recited in any one of claims 8 to 12, wherein the miRNA is a miRNA derivative comprising at least one modified internucleoside linkage, at least one modified sugar moiety, at least one modified base, or any combinations thereof.
